# EUROPÄISCHE PATENTSCHRIFT

(11) **EP 0 868 902 B1**
(45) Veröffentlichungstag und Bekanntmachung des Hinweises auf die Patenterteilung: **12.05.2004**
(21) Anmeldenummer: 98105194.9
(22) Anmeldetag: 23.03.1998
(51) Int. Cl.: A61K 7/13

(54) **Haarfärbemittel**
Hair dye
Composition de teinture pour cheveux

(30) Priorität: 01.04.1997 DE 19713510
(43) Veröffentlichungstag der Anmeldung: 07.10.1998
(73) Patentinhaber: Henkel Kommanditgesellschaft auf Aktien, 40589 Düsseldorf-Holthausen (DE)
(72) Erfinder: Höffkes, Horst, Dr. Dipl.-Chem., 40595 Düsseldorf (DE); Büttner, Roswitha, 4077 Düsseldorf (DE)

(56) Entgegenhaltungen:
- GB-A- 2 119 411
- GB-A- 2 187 210
- US-A- 5 190 564
- US-A- 5 275 626

## Beschreibung

Die Erfindung betifft Haarfärbemittelzubereitungen, die öllösliche oder in Wasser instabile Farbstoffe in einem flüssigen, wasserfreien Träger enthalten, sowie Haarfärbemittel, die aus wenigstens zwei getrennt verpackten, zu einer Verkaufseinheit zusammengefaßten Zubereitungen bestehen, die unmittelbar vor der Anwendung miteinander gemischt werden und die als eine erste Zubereitung eine solche wasserfreie Haarfärbemittelzubereitung enthalten.

Es sind zahlreiche direktziehende Haarfarbstoffe bekannt, darunter auch solche, die in Wasser schwer löslich oder instabil sind und daher aus wäßriger Phase nur schwache Färbungen ergeben. Für solche Farbstoffe hat man auch schon wasserfreie Formulierungen vorgeschlagen, zum Beispiel in Form von trockenen Pulvern, die als Kataplasma angewendet oder die erst kurz vor ihrer Anwendung in Wasser gelöst werden.

Für schwer wasserlösliche Chinonfarbstoffe wurden in DE 33 15 522 A1 bereits wasser-freie Lösungen in organischen Lösungsmitteln vorgeschlagen, die auch einen oberflächenaktiven Stoff enthalten. Es hat sich jedoch gezeigt, daß solche Lösungen, wenn sie mit wäßrigen Zubereitungen, z.B. von wasserlöslichen Farbstoffen oder von Oxidationsmitteln, vermischt werden, dem Färbeansatz nicht die geeignete Konsistenz verleihen, die für eine Anfärbung der Haare erforderlich ist.

Die Patentschrift GB 2 119 411 A offenbart eine lagerstabile Zusammensetzung zur Haarfärbung enthaltend (a) 0.001-5% Farbstoff, (b) eine Ölkomponente, z.B. mehr als 15% eines synthetischen Fettsäureester mit einem Polyol, (c) mehr als 20% eines nichtionischen polyethoxylierten Fettalkohols und (d) ein zusätzlicher Verdicker. Allerdings schweigt sich dieses Schrift über die Menge und die chemische Beschaffenheit des Verdickers aus.

Die Patentschriften US 5 275 626 und US 5 190 564 beschreiben Haarfärbeverfahren in welchem (a) 0.05-5% eines Monohydroxyindolderivats und 0.01-5% eines aromatischen Carbonylderivates in einem nicht-wässrigen Medium vorliegen, welches wiederum aus mindestens 15% eines nicht-wässrigen Lösungsmittel, mindestens 20% eines Tensids und einem Verdicker besteht. Ausserdem wird ein Kit aus verschiedenen Abteilen beschrieben, wobei die getrennt verpackten Zubereitungen nicht-wässrig bzw. wässrig oder nur wässrig sein können. Das nichtionische Tensid und der Verdicker müssen vom Fachmann von zwei unabhängigen Listen ausgewählt werden. Die Menge und die chemische Beschaffenheit des Verdickers sind nicht offenbart.

Aus WO 95/24886 sind Isatinderivate bekannt, die sich zur Färbung von Haaren eignen, wobei besonders tiefe und brillante Nuancen erhalten werden, wenn gleichzeitig aminogruppenhaltige Verbindungen, z.B. Aminosäuren, Oligopeptide und insbesondere aromatische Aminoverbindungen auf das Haar einwirken. Unter den aromatischen und heterocyclischen Aminoverbindungen sind auch Verbindungen, die bereits als Oxidationsfärbemittel-Vorstufen bekannt sind, z.B. p-Toluylendiamin, p-Aminophenole oder Tetraamino-pyrimidine. Aus der deutschen Auslegeschrift DE 29 32 489 sind aromatische Aldehyde und aus der deutschen Patentanmeldung DE 196 30 275.7 sind vinyloge, aromatische Aldehyde bekannt, die zusammen mit z.B. aromatischen Aminoverbindungen unter Mitwirkung eines Oxidationsmittels Haarfärbungen erzeugen. In den genannten Druckschriften sind zahlreiche Verbindungen beschrieben, die nur eine begrenzte Wasserlöslichkeit von weniger als 1 g/l (20° C) aufweisen und daher - auch in Verbindung mit den aromatischen Aminoverbindungen - nur einen schwachen Farbaufzug auf das Haar bewirken.

Es wurde nun eine wasserfreie, flüssige Haarfärbemittelzubereitung gefunden, die auch nach Mischung mit wäßrigen Zubereitungen, z.B. von wasserlöslichen Farbstoffen oder Farbstoffvorprodukten oder von Oxidationsmitteln oder beidem, einen Haarfärbeansatz von befriedigender Konsistenz und sehr guter Färbekraft liefert.

Gegenstand der Erfindung ist daher eine Haarfärbemittelzubereitung, bestehend aus einem wasserfreien Träger und darin gelösten Farbstoffen, wobei als Farbstoffe 0,15 bis 15 Gew.-% öllösliche oder instabile Direktfarbstoffe und als Trägerkomponenten
25 - 90 Gew.-% wasserunlösliche flüssige Ölkomponenten,
2 - 7 Gew.-% nichtionische Emulgatoren mit einem HLB-Wert von 5 oder mehr,
3 - 50 Gew.-% Verdickungsmittel, ausgewählt aus einer Gruppe, bestehend aus C₁₆-C₁₈-Fettsäurepartialestern von C₂-C₆-Polyolen mit 2 bis 6 Hydroxylgruppen und C₁₆-C₁₈-Fettsäure-Metallseifen sowie gegebenenfalls
0 - 50 Gew.-% wasserlösliche Polyole oder Polyethylenglycole
enthalten sind.

Als wasserfrei im Sinne der vorliegenden Erfindung werden Zusammensetzungen mit einem Wassergehalt unter 1 Gew.-% verstanden. Als öllösliche Farbstoffe werden solche Farbstoffe verstanden, deren Löslichkeit in Paraffinöl bei 20° C oberhalb 0,1 Gew.-% liegt. Als instabile Farbstoffe werden solche verstanden, die bei Lagerung bei 20° C in Wasser eine chemische Veränderung erleiden, so daß nach 8 Wochen Lagerung weniger als 80 % der ursprünglichen Färbewirkung erhalten bleibt.

Flüssige Ölkomponenten im Sinne der vorliegenden Erfindung sind alle physiologisch verträglichen, bei 20° C flüssigen mineralischen, tierischen, pflanzlichen oder synthetischen Ölkomponenten. Beispiele für solche Ölkomponenten sind z.B. Paraffinöle, Silikonöle, Triglyceridöle, z.B. Klauenöl, Lardöl, Nerzöl, Olivenöl, Sonnenblumenöl, Mandelöl, flüssige Wachsester wie z.B. Spermöl, Jojobaöl, synthetische Ester wie z.B. Glycerin-tricaprylat, n-Hexyllaurat, Isopropylmyristat, 2-Ethylhexyl-stearat, Butyloleat, synthetische Ether wie z.B. Di-n-octylether, synthetische Kohlenwasserstoffe wie z.B. Diisooctyl-cyclohexan, Squalan, synthetische Alkohole wie z.B. 2-Octyl-dodecanol oder 2-Hexyl-decanol.

Nichtionische Emulgatoren im Sinne der Erfindung sind alle in den Ölkomponenten löslichen nichtionogenen oberflächenaktiven Verbindungen, die eine lipophile, bevorzugt lineare Alkyl- oder Acylgruppe mit 10 - 22 C-Atomen und einen hydrophilen Molekülteil, z.B. in Form eines Polyol- oder Polyetherrestes aufweisen, wobei der HLB-Wert im Bereich von 5 oder darüber liegen sollte.

Unter dem HLB-Wert wird dabei der Wert verstanden, der sich aus der Beziehung HLB = 0,2 • (100 - L) ergibt; dabei ist L der Gewichtsanteil der lipophilen Alkyl- oder Acylgruppen (in Gew.-%) im Molekül.

Geeignete nichtionische Emulgatoren sind z.B. die Anlagerungsverbindungen von 2 bis 8 Mol Ethylenoxid an lineare Fettalkohole mit 12 bis 18 C-Atomen, an Fettsäuremonoglyceride oder an Sorbitanfettsäuremonoester. Weitere geeignete nichtionische Ten-side sind Alkyl-(oligo)-glycoside und deren Oxethylate, die Anlagerungsverbindungen von Ethylenoxid an Methylglucosid-Fettsäureester sowie Polyglycerinderivate (z.B. Poly- glycerin-poly-12-hydroxystearat).

Geeignete Verdickungsmittel vom Typ der C₁₆-C₁₈-Fettsäurepartialester von C₂-C₆-Polyolen sind z.B. Fettsäurepartialglyceride und Sorbitanfettsäure-partialester. Unter Fettsäure-Partialestern werden dabei bevorzugt die Monoester und Glycolen und die Mono- und Diester von drei- und mehrwertigen Polyolen verstanden.

Besonders bevorzugte Haarfärbemittelzubereitungen enthalten als Verdickungsmittel ein C₁₆-C₂₂-Fettsäure-mono- oder -diglycerid, eine C₁₆-C₂₂-Fettsäure-, Alkali-, Erdalkalioder Aluminiumseife oder ein Gemisch davon.

Handelsübliches Glycerinmonostearat (GMS) ist ein technisches Gemisch aus ca. 45 Gew.-% Monoglycerid, 40 Gew.-% Diglycerid, 10 Gew.-% Triglycerid und bis 5 Gew.-% freiem Glycerin.

Weitere Verdickungsmittel vom Typ der C₁₂-C₂₂-Fettsäure-Metallseifen sind z.B. die öllöslichen Seifen der Palmitinsäure, der Stearinsäure, der Isostearinsäure oder der Ölsäure.

Eine handelsübliche Metallseife ist z.B. das Magnesium-Aluminium-hydroxy-stearat, das in verschiedenen Ölkomponenten gelöst (ca. 20 Gew.-%) als Gilugel® (Giulini Chemie) angeboten wird. Die Herstellung solcher Geliermittel ist z.B. in EP 0 419 759 B1 be-schrieben.

In einer bevorzugten Ausführungsform enthält die Haarfärbemittelzubereitung 0,5 - 8 Gew.-% Farbstoffe, 30 - 80 Gew.-% wasserlöslicher flüssiger Ölkomponenten, 5 - 50 Gew.-% nichtionischer Emulgatoren mit HLB-Werten von 5 bis 15 und 5 - 20 Gew.-% eines C₁₆-C₂₂-Fettsäuremonoglycerids oder eines Magnesium-Aluminium-Hydroxystearats.

Zur Steuerung der Viskosität der wasserfreien Träger und zur Verhinderung eines Verlustes der Fließfähigkeit kann man - falls erforderlich - niedermolekulare Polyole mit 2 bis 6 C-Atomen und 2 bis 6 Hydroxylgruppen oder Polyethylenglycole zusetzen. Geeignete Polyole sind z.B. 1,2-Propylenglycol, Glycerin, Sorbit, Diethylenglycol, Dipropylenglycol oder ein Polyethylenglycol mit dem mittleren Molgewicht von 200 - 600.

Als Farbstoffe können prinzipiell alle in Ölen löslichen Farbstoffe, die sich zur Färbung von Keratinfasern eignen, eingesetzt werden. Bevorzugt werden aber solche verwendet, die sich in Wasser nur wenig lösen oder in wäßrigen Zubereitungen nicht lagerstabil sind.

Von besonderem anwendungstechnischem Interesse sind die erfindungsgemäßen Haarfärbemittelzubereitungen, wenn als Farbstoffe solche aus der Gruppe der Isatinderivate oder der aromatischen oder vinylogen Carbonylverbindungen enthalten sind, da gerade diese Farbstoffe in Wasser oft schwer löslich sind und aus wäßrigen Zubereitungen nur schwache Färbungen ergeben.

Ganz besonders bevorzugte Farbstoffe, die in ihrer Färbekraft durch die erfmdungsgemäßen Haarfärbemittelzubereitungen besonders intensiviert werden, sind z.B. das 1-Allylisatin, das 1-Diethylaminomethylisatin, das 1-Diethylaminomethylisatin, das 1-Piperidinomethylisatin, der 4-Hydroxy-3-methoxyzimtaldehyd, das Glutaconaldehydtetra-butylammoniumsalz sowie der 2-(1,3,3,-Trimethyl-2-indolyliden)-acetaldehyd.

Andererseits lassen sich Farbintensität und Nuancenumfang dieser Farbstoffe durch Kombination mit wasserlöslichen aromatischen oder heterocyclischen Aminen oder Phenolen oder auch mit anderen wasserlöslichen Aminoverbindungen, z.B. mit Aminosäuren, deutlich steigern.

Ein besonders bevorzugtes Verfahren der Haaranfärbung besteht nun darin, daß man eine erfindungsgemäße wasserfreie Haarfärbemittelzubereitung mit einer wäßrigen Zubereitung eines wasserlöslichen Farbstoffs oder Farbstoffvorprodukts mischt und die sich dabei bildende Emulsion auf das Haar bringt. Auf diese Weise wird eine besonders starke Intensivierung des Farbaufzugs und der Farbtiefe erreicht.

Besonders günstige Ergebnisse werden in solchen Fällen erzielt, in denen als wasserlöslicher Farbstoff eine Oxidationsfarbstoff-Entwicklerverbindung, z.B. ein p-Toluylendiamin, ein p-Aminophenol oder 2,4,5,6-Tetraaminopyrimidin verwendet wird. Eine weitere Möglichkeit zur Erweiterung der Nuancenvielfalt besteht darin, als wasserlösliche Farbstoffe ein Gemisch von Entwickler- und Kupplerverbindungen einzusetzen und dem Färbeansatz eine weitere Zubereitung eines Oxidationsmittels, z.B. von Wasserstoffperoxid, dem Färbeansatz zuzumischen.

Ein weiterer bevorzugter Gegenstand der Erfindung ist daher ein Haarfärbemittel, bestehend aus wenigstens zwei, getrennt verpackten, in einer Verkaufseinheit (Kit) zusammengefaßten Zubereitungen, die unmittelbar vor der Anwendung miteinander gemischt werden, wobei als eine erste Zubereitung (A) eine wasserfreie Haarfärbemittelzubereitung (A) gemäß der Erfindung und als eine zweite Zubereitung (B) eine wäßrige Zubereitung von wasserlöslichen Farbstoffen oder Farbstoffvorprodukten, bevorzugt von wasserlöslichen aromatischen oder heterocyclischen Aminen oder Phenolen oder deren Salzen enthalten ist.

Es kann dabei erwünscht und anwendungstechnisch vorteilhaft sein, wenn diese Haarfärbemittel eine dritte - ebenfalls getrennt verpackte - Zubereitung (C) mit einem Oxidationsmittel enthalten.

Die Oxidationsmittelzubereitung (C) kann entweder wäßrig, z.B. im Falle von Wasserstoffperoxid sein, sie kann aber auch in Form eines wasserfreien Pulvers, z.B. eines Wasserstoffperoxid-Addukts an Harnstoff (Percarbamid) oder an Melamin (Melaminperhydrat) oder in Form einer anderen Perverbindung, z.B. Magnesiumperoxid oder Kaliumpersulfat vorliegen.

Zusätzlich zu den genannten Komponenten kann die erfindungsgemäße wasserfreie Haar-färbemittelzubereitung (A) noch weitere Hilfsmittel in untergeordneten Mengen, z.B. Duftstoffe, Antioxidantien oder öllösliche haarkosmetische Wirkstoffe enthalten.

Die wäßrigen Zubereitungen (B) der wasserlöslichen Farbstoffe oder Farbstoffvorprodukte können z.B. ebenfalls oberflächenaktive Substanzen, z.B. anionische, zwitterionisehe oder nichtionische Tenside, wasserlösliche polymere Verdickungsmittel, Antioxidantien, Puffersalze, emulgierte Fett- oder Ölkomponenten oder Gemische solcher Zusätze enthalten. Darüber hinaus können haarkosmetische Wirkstoffe, z.B. Antischuppenmittel, Avivagemittel oder Festiger darin enthalten sein. Für das Färbeergebnis kann es vorteilhaft sein, wenn zusätzlich wasserlösliche Salze von Metallen, z.B. von Alkalimetallen, Erdalkalimetallen, Zink oder Kupfer darin enthalten sind.

Die Herstellung der erfindungsgemäßen wasserfreien Haarfärbemittelzubereitungen (A) erfolgt bevorzugt in der Weise, daß man zuerst den Träger durch Vermischen der Komponenten, gegebenenfalls bei einer Temperatur bis zu 100° C, herstellt und nach dem Abkühlen auf Umgebungstemperatur den öllöslichen Farbstoff darin löst.

Zur Herstellung der gebrauchsfertigen Färbezubereitung wird erst unmittelbar, also nicht mehr als 10 Minuten vor der eigentlichen Haarfärbung, die erfindungsgemäße flüssige, wasserfreie Haarfärbemittelzubereitung (A) mit einer zweiten wäßrigen Zubereitung (B) und ggf. mit einer weiteren Zubereitung (C) vermischt. Der dabei erhaltene Haarfärbeansatz sollte eine noch fließfähige, aber doch so hohe Konsistenz haben, daß er mit einer Haarfärbebürste auf das Haar aufgetragen werden kann und nicht vom Haar herabtropft. Dieses rheologische Verhalten wir durch die erfindungsgemäßen Färbemittelzuberei-tungen besonders gut erreicht. Es kann auch vorteilhaft sein, die Komponenten A, B und C nacheinander anzuwenden. Dabei wird bevorzugt erst die wäßrige Komponente B appliziert und danach die Komponente A oder gegebenenfalls das Gemisch aus Komponente A und Komponente C auf das Haar aufgebracht.

Die folgenden Beispiele sollen den Gegenstand der Erfindung näher erläutern:

### Beispiele

### 1. Herstellung wasserfreier Träger

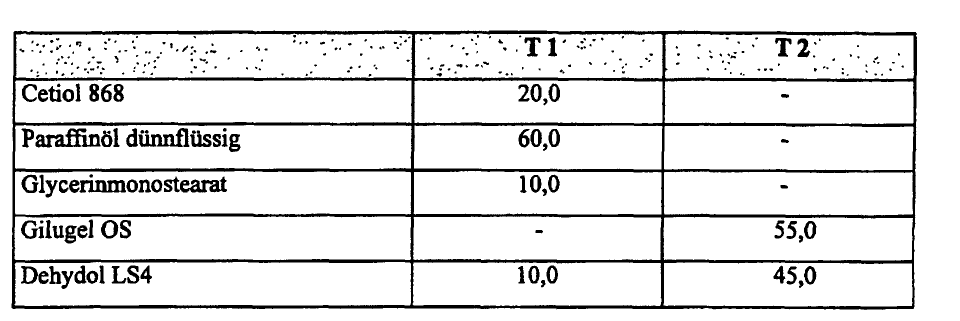

### Herstellung:

- Träger 1 :: Die Komponenten werden gemischt und auf 90°C erwärmt, dann auf 20°C gekühlt.
- Träger 2 :: Die Komponenten werden kalt gemischt.

### 2. Herstellung wäßriger Träger

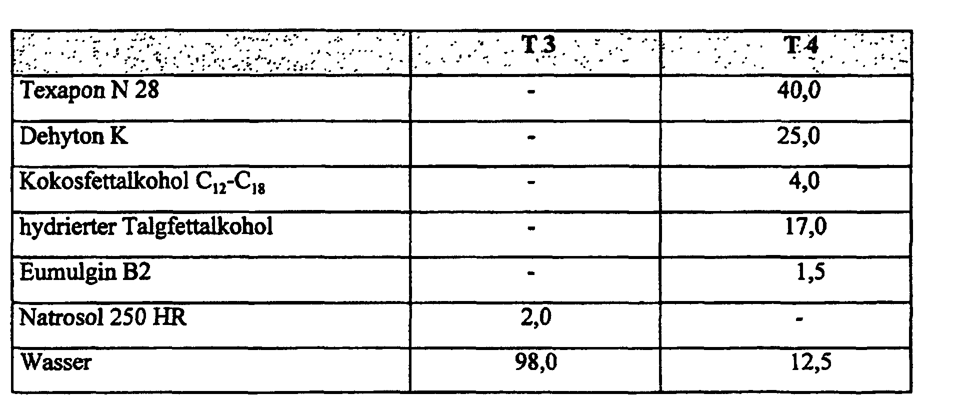

### Herstellung:

- Träger 3 :: Die Quellung wird bei 60°C hergestellt, dann auf 20°C gekühlt.
- Träger 4 :: Fettalkohole und Eumulgin B2 werden geschmolzen, die übrigen Komponenten in Wasser gelöst, auf 70°C erwärmt und unter Rühren zur Schmelze gegeben. Nach Emulsionsbildung wird unter Rühren auf 20°C gekühlt.

### 3. Herstellung von Färbemittelkomponenten

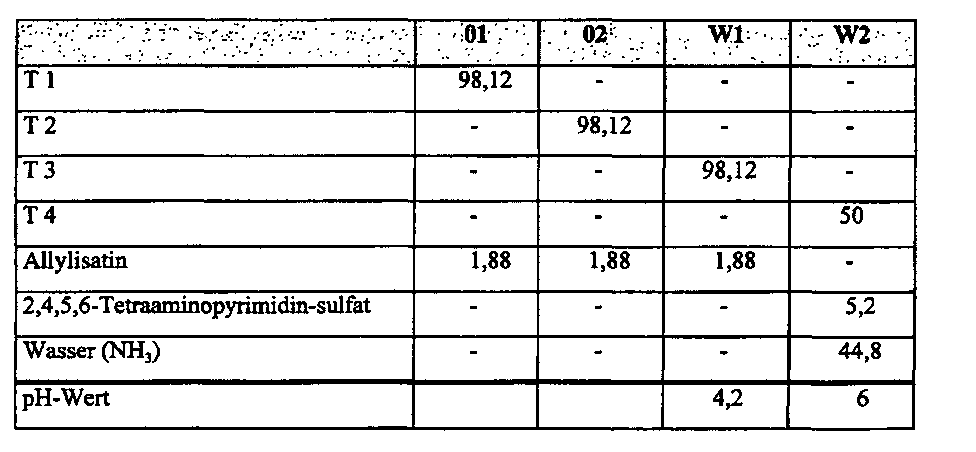

### 4. Herstellung der Haarfärbemittel und Ausfärbung

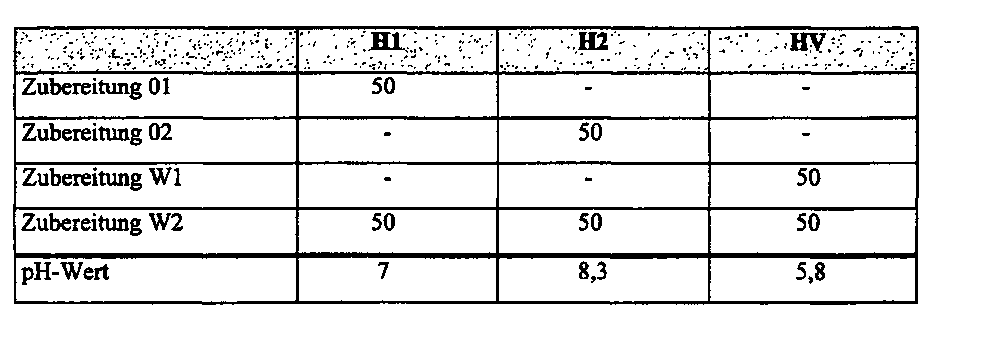

Je 4 ml der frisch hergestellten Färbemischungen H1, H2 und HV wurden auf Strähnen von 1 g blondem Humanhaar aufgetragen. Die Strähnen wurden mit Al-Folie umwickelt und 30 Minuten bei 32°C im Thermostaten (Umlufttrockenschrank) gelagert.

Die Färbemischungen H1 und H2 lieferten intensiv tizianrot gefärbte Haare. Die Färbemischung HV lieferte hingegen nur schwach orangegelb gefärbte Haare.

Es wurden die folgenden Rohstoffe verwendet:
- Cetiol®868 :: 2-Ethylhexyl-stearat
- Gilugel®OS :: Al-Mg-Hydroxystearat (20 Gew.-%) in 2-Ethylhexylstearat
- Dehydol®LS4 :: C_{12/14}-Fettalkohol + 4 EO
- Eumulgin®B2 :: C_{16/18}-Fettalkohol + 20 EO
- Texapon®N28 :: C_{12/14}-Fettalkohol-(2 EO)-ethersulfat, Na-Salz (28 %ig in Wasser)
- Dehyton ®K :: Cocoamidopropyl-Betaine (30 %ige Lösung in Wasser) (N-C_{8/18}-Kokosacylamidopropyl-dimethyl-acetobetain)
- Natrosol®250 HR :: Hydroxyethylcellulose
Viskosität (1 % in H₂O) : 1,5 - 2,5 Pa·s (20°C)
Viskosität (2 % in H₂O) : 30 Pa·s (20°C)

## Patentansprüche

1. Haarfärbemittelzubereitung, bestehend aus einem wasserfreien Träger und darin gelösten Farbstoffen, **dadurch gekennzeichnet, daß** als Farbstoffe 0,1 bis 15 Gew.-% öllösliche oder in Wasser instabile Direktfarbstoffe und als Trägerkomponenten
25 - 90 Gew.-% wasserunlösliche flüssige Ölkomponenten
2 - 70 Gew.-% nichtionische Emulgatoren mit HLB-Werten von 5 und darüber
3 - 50 Gew.-% Verdickungsmittel, ausgewählt aus der Gruppe, bestehend aus C₁₆-C₁₈-Fettsäurepartialestern von C₂-C₆-Polyolen mit 2 - 6 Hydroxylgruppen und C₁₆-C₁₈-Fettsäure-Metallseifen sowie gegebenenfalls
0 - 50 Gew.-% wasserlösliche Polyole oder Polyethylenglycole
enthalten sind.

2. Haarfärbemittelzubereitung nach Anspruch 1, **dadurch gekennzeichnet, daß** als Verdickungsmittel ein C₁₆-C₂₂-Fettsäuremono- oder -diglycerid, eine C₁₆-C₂₂-Fettsäure-, Alkali-, Erdalkali- oder Aluminiumseife oder ein Gemisch davon enthalten ist.

3. Haarfärbemittelzubereitung nach einem der Ansprüche 1 bis 2, **dadurch gekennzeichnet, daß**
0,5 - 8 Gew.-% Farbstoffe und
30 - 80 Gew.-% wasserunlöslicher flüssiger Ölkomponenten,
5 - 50 Gew.-% nichtionischer Emulgatoren mit HLB-Werten von 5 bis 15 und
5 - 20 Gew.-% eines C₁₆-C₂₂-Fettsäuremonoglycerids oder eines Magnesium-Aluminium-Hydroxystearats
enthalten sind.

4. Haarfärbemittelzubereitung nach einem der Ansprüche 1 bis 3, **dadurch gekennzeichnet, daß** als Farbstoffe solche aus der Gruppe Isatinderivate oder der aromatischen oder vinylogen Carbonylverbindungen enthalten sind.

5. Haarfärbemittel-kit, bestehend aus wenigstens zwei getrennt verpackten, zu einer Verkaufseinheit zusammengefaßten Zubereitungen, die unmittelbar vor der Anwendung miteinander gemischt werden, **dadurch gekennzeichnet, daß** als eine erste Zubereitung (A) eine wasserfreie Haarfärbemittelzubereitung gemäß einem der Ansprüche 1 bis 4 und als eine zweite Zubereitung (B) eine wäßrige Zubereitung von wasserlöslichen Farbstoffen oder Farbstoffvorprodukten, bevorzugt von wasserlöslichen aromatischen oder heterocyclischen Aminen oder Phenolen oder deren Salzen, enthalten ist

6. Haarfärbemittel-kit nach Anspruch 5, **dadurch gekennzeichnet, daß** eine dritte - getrennt verpackte - Zubereitung mit einem Gehalt an einem Oxidationsmittel enthalten ist.

## Claims

1. A hair colouring preparation consisting of a water-free carrier and dyes dissolved therein, **characterized in that** it contains 0.1 to 15% by weight oil-soluble direct dyes or direct dyes unstable in water as the dyes and, as carrier components,
25 to 90% by weight water-insoluble liquid oil components,
2 to 70% by weight nonionic emulsifiers with HLB values of 5 or higher,
3 to 50% by weight thickeners selected from the group consisting of C₁₆₋₁₈ fatty acid partial esters of C₂₋₆ polyols containing 2 to 6 hydroxyl groups and C₁₆₋₁₈ fatty acid metal soaps and optionally
0 to 50% by weight water-soluble polyols or polyethylene glycols.

2. A hair colouring preparation as claimed in claim 1, **characterized in that** it contains a C₁₆₋₂₂ fatty acid mono- or diglyceride, a C₁₆₋₂₂ fatty acid, alkali metal, alkaline earth metal or aluminium soap or a mixture thereof as the thickener.

3. A hair colouring preparation as claimed in claim 1 or 2, **characterized in that** it contains
0.5 to 8% by weight dyes,
30 to 80% by weight water-insoluble liquid oil components,
5 to 50% by weight nonionic emulsifiers with HLB values of 5 to 15 and
5 to 20% by weight of a C₁₆₋₂₂ fatty acid monoglyceride or a magnesium aluminium hydroxystearate.

4. A hair colouring preparation as claimed in any of claims 1 to 3, **characterized in that** the dyes present are dyes from the group of isatin derivatives or aromatic or vinylogous carbonyl compounds.

5. A hair colouring kit consisting of at least two separately packed preparations combined to form a retail article which are mixed together immediately before use, **characterized in that** the water-free hair colouring preparation claimed in any of claims 1 to 4 is present as a first preparation (A) and an aqueous preparation of water-soluble dyes or dye precursors, preferably water-soluble aromatic or heterocyclic amines or phenols or salts thereof, is present as a second preparation (B).

6. A hair colouring kit as claimed in claim 5, **characterized in that** a third - separately packed - preparation containing an oxidizing agent is present.

## Revendications

1. Préparation de teinture capillaire, se composant d'un véhicule anhydre et de colorants dissous dans celui-ci, **caractérisée en ce que** 0,1 à 15% en poids de colorants directs, liposolubles ou instables dans de l'eau, y sont contenus en tant que colorants et
25 à 90% en poids de composants d'huile liquides insolubles dans l'eau,
2 à 70% en poids d'émulsifiants non ioniques ayant des valeurs d'équilibre hydrophile-lipophile (HLB) de 5 et de plus
3 à 50% en poids d'épaississant, choisi dans le groupe constitué par les esters partiels d'acides gras en C₁₆ à C₁₈ de polyols en C₂ à C₆, avec 2 à 6 groupes hydroxyles et des savons métalliques d'acides gras en C₁₆ à C₁₈, ainsi qu'éventuellement
0 à 50% en poids de polyols ou polyéthylèneglycols hydrosolubles,
y sont contenus en tant que composants de véhicule.

2. Préparation de teinture capillaire selon la revendication 1, **caractérisée en ce qu'**un mono- ou di-glycéride d'acide gras en C₁₆ à C₂₂, un savon d'acide gras en C₁₆ à C₂₂, de métal alcalin, de métal alcalino-terreux ou d'aluminium, ou un mélange de ceux-ci, y sont contenus en tant qu'épaississants.

3. Préparation de teinture capillaire selon l'une quelconque des revendications 1 à 2, **caractérisée en ce que**,
0,5 à 8% en poids de colorants et
30 à 80% en poids de composants d'huile liquides insolubles dans l'eau,
5 à 50% en poids d'émulsifiants non ioniques avec des valeurs HLB de 5 à 15 et
5 à 20% en poids d'un monoglycéride d'acide gras en C₁₆ à C₂₂, ou un hydroxystéarate de magnésium-aluminium
y sont contenus.

4. Préparation de teinture capillaire selon l'une quelconque des revendications 1 à 3, **caractérisée en ce qu'**en tant que colorants, ceux provenant du groupe des dérivés de l'isatine ou des composés carbonyles aromatiques ou vinylogues y sont contenus.

5. Nécessaire de colorant capillaire, se composant d'au moins deux préparations empaquetées de façon séparée, réunies en une unité de vente, qui sont mélangées l'une à l'autre directement avant l'application, **caractérisé en ce qu'**une préparation de teinture capillaire anhydre selon l'une quelconque des revendications 1 à 4, en tant que première préparation (A) et une préparation aqueuse de colorants ou de précurseurs de colorants, hydrosolubles, de préférence d'amines ou de phénols hydrosolubles, aromatiques ou hétérocycliques, ou leurs sels, en tant que deuxième préparation (B), y sont contenues.

6. Nécessaire de teinture capillaire selon la revendication 5, **caractérisé en ce qu'**une troisième préparation empaquetée de façon séparée, ayant une teneur en un agent d'oxydation, y est contenue.
